# EUROPEAN PATENT SPECIFICATION

(11) **EP 2 209 481 B1**
(45) Date of publication and mention of the grant of the patent: **13.03.2013**
(21) Application number: 07827768.8
(22) Date of filing: 15.10.2007
(51) Int. Cl.: A61K 35/14, C12N 5/0789, A61K 35/39, A61P 37/06

(54) **USE OF MICROVESICLES (MVS) FOR PREPARING A MEDICAMENT HAVING ADJUVANT ACTIVITY ON ENDOTHELIAL CELL TRANSPLANTATION, PARTICULARLY IN THE TREATMENT OF DIABETES BY PANCREATIC ISLET TRANSPLANTATION, AND RELATED METHOD**
VERWENDUNG VON MIKROVESIKELN (MVS) ZUR HERSTELLUNG EINES MEDIKAMENTS MIT ADJUVANTER WIRKUNG AUF ENDOTHELZELLEN-TRANSPLANTATION, INSBESONDERE BEI DER BEHANDLUNG VON DIABETES DURCH PANKREATISCHE INSELZELL-TRANSPLANTATION UND RELEVANTES VERFAHREN
UTILISATION DE MICROVÉSICULES (MVS) POUR PRÉPARER UN MÉDICAMENT AYANT UNE ACTIVITÉ D'ADJUVANT SUR LA TRANSPLANTATION DE CELLULES ENDOTHÉLIALES, EN PARTICULIER DANS LE TRAITEMENT DU DIABÈTE PAR UNE TRANSPLANTATION D'ÎLOTS PANCRÉATIQUES, ET PROCÉDÉ ASSOCIÉ

(43) Date of publication of application: 28.07.2010
(73) Proprietor: Fresenius Medical Care Deutschland GmbH, 61352 Bad Homburg (DE)
(72) Inventor: CANTALUPPI, Vincenzo, I-22038 Tavernerio (Como) (IT); DEREGIBUS, Maria Chiara Amilde, I-10132 Torino (IT); CAMUSSI, Giovanni, I-10132 Torino (IT)
(74) Representative: Comoglio, Elena
(86) International application number: PCT/IT2007/000717
(87) International publication number: WO 2009/050742

(56) References cited:
- WO-A-2005/121369
- DEREGIBUS MARIA CHIARA ET AL: "Endothelial progenitor cell derived microvesicles activate an angiogenic program in endothelial cells by a horizontal transfer of mRNA" BLOOD, W.B.SAUNDERS COMPANY, ORLANDO, FL, vol. 110, no. 7, 1 October 2007 (2007-10-01), pages 2440-2448, XP009101333 ISSN: 0006-4971
- BRISSOVA MARCELA ET AL: "Intraislet endothelial cells contribute to revascularization of transplanted pancreatic islets." DIABETES MAY 2004, vol. 53, no. 5, May 2004 (2004-05), pages 1318-1325, XP002486789 ISSN: 0012-1797
- MATHEWS VIKRAM ET AL: "Recruitment of bone marrow-derived endothelial cells to sites of pancreatic beta-cell injury." DIABETES JAN 2004, vol. 53, no. 1, January 2004 (2004-01), pages 91-98, XP002486790 ISSN: 0012-1797
- SHAPIRO A M ET AL: "Islet transplantation in seven patients with type 1 diabetes mellitus using a glucocorticoid-free immunosuppressive regimen" NEW ENGLAND JOURNAL OF MEDICINE, THE, MASSACHUSETTS MEDICAL SOCIETY, WALTHAM, MA, US, vol. 343, no. 4, 27 July 2000 (2000-07-27), pages 230-238, XP002220559 ISSN: 0028-4793

## Description

The present invention generally falls within the field of the endothelial cell-pancreatic islet transplantation, and in particular it relates to the therapeutic treatment of type I and II diabetes by pancreatic islet transplantation.

In the last years, islet transplantation has become a rising therapeutic option for the treatment of type I and type II diabetes after the introduction of a rapamycin-based glucocorticoid-free immunosuppressive regimen and the improvement of isolation techniques (1-3). However, a great percentage of transplanted islets still fails to engraft into the liver after portal vein infusion and, as a consequence, pancreata from multiple donors are necessary to guarantee a sufficient islet mass to achieve a metabolic benefit.

In order to increase the success of the procedure, it would be necessary to identify factors which capable of enhancing the functionality and survival of transplanted islets.

Endothelial progenitor cells (EPCs) are known to be recruited to the pancreas in response to islet injury and it is also known that EPC-mediated pancreas neovasculatization may facilitate the recovery of injured β-cells improving islet allograft function (4,5). However, the use of EPCs in cellular transplant is not advisable given the potential tumorigenic risk of stem cells.

The references Brissova Marcela et al.: "Intraislet endothelial cells contribute to revascularization of transplanted pancreatic islets", Diabetes, May 2004, vol. 53, No. 5, pages 1318-1325 and Mathews Virkram et al. "Recruitment of bone marrow-derived endothelial cells to sites of pancreatic beta-cell injury", Diabetes, January 2004, vol. 53, No. 1, pages 91-98, disclose the use of pancreatic islet transplantation in the treatment of type I diabetes, and reveal that a co-transplantation with exogenous endothelial cells or EPCs, may promote islet engraftment and revascularization.

The present invention have now found that microvesicles (MVs) derived from cells of the endothelial cell lineage, specifically from endothelial progenitor cells (EPCs), represent an advantageous alternative over the whole stem cells as an adjuvant factor in the type I and type II diabetes therapy by islet transplantation.

The expression "microvesicles (MVs) derived from cells of the endothelial cell lineage" as used herein refers to a membranaceous particle which is at least in part derived from the endosomal compartment of a cell of the endothelial cell lineage upon fusion with the outer Cell membrane, specifically from the endosomal compartment of a EPC.

Microvesicles derived from cells of the endothelial cell lineage, specifically from EPCs, are generally spheroid in shape and have a diameter within the range of 100 nm to 5 µm, more typically of about I µm. If the particle is not spheroid in shape, the above-mentioned values are referred to the largest dimension of the particle.

The expression "cells of the endothelial lineage" refers to cells which derive from common hematopoietic precursors originated in the bone marrow able to differentiate into mature functional endothelial cells (6).

The cells of the endothelial lineage, specifically EPCs, are conveniently isolated from peripheral blood by density centrifugation and plated on a culture medium such as EBM-2 *(endothelial basal medium*)*,* supplemented with endothelial growth factors (Deregibus M C et al., Blood. 1 Oct 2007; 110(7):2440-8. Pre-published online on 29 May 2007). Microvesicles (MVs) may then be obtained from the supernatants of the isolated EPCs, by ultracentrifugation techniques as disclosed in Deregibus, 2007 and in the experimental section of the present description.

Isolated MVs may then be stored until use by freezing at very low temperature, typically at -80°C, in a suspension with one or more cryoprotecting agents. Suitable cryoprotecting substances are for example dimethylsulphoxide (DMSO) and glycerol. The use of DMSO at a concentration of 10% of the cell suspension volume guarantees good preservation of the cells and a limited toxic effect on reinfused patients. Other substances which may be cited are extracellular cryoprotecting agents, that is to say high molecular weight substances acting at the cell surface forming a tight barrier which reduces intracellular dehydration. Hydroxyethylic starch may be cited as an example.

EPC-derived MVs were tested by the present inventors both *in vitro* and in *vivo* in an experimental model of subcutaneous islet transplantation in SCID mice. SCID mice are not capable of producing T and B cells and as a consequence they are not capable of fighting infections and of rejecting transplanted tissue.

The experimental work carried out by the present inventors, which is illustrated in further detail in the experimental section of the description, showed that EPC-derived MVs, when administered to an endothelial cell-pancreatic islet transplant recipient, and particularly to a pancreatic islet transplant recipient, act as an adjuvant factor for transplant, in that they improve the survival and functionality of transplanted endothelial cells. More particularly, the inventors observed that MVs are capable of promoting angiogenesis and capillary-like structures formation from endothelial cells, as well the secretion of insulin from islet β-cells as well as the replication, resistance to apoptosis and migration of endothelial cells. Most importantly, the above-mentioned adjuvant effect of MVs is not entirely inhibited by incubation with therapeutic doses of rapamycin, the basic immunosuppressant in pancreatic islet transplantation. This is most surprising since rapamycin is known to exert a dual effect on islet endothelium, with the induction of a simultaneous immunomodulatory effect through the down-regulation of receptors involved in lymphocyte adhesion and activation, but also the inhibition of angiogenesis (7).

Therefore, one of the invention is the use of microvesicles (MVs) derived from an endothelial progenitor cell (EPC), for preparing a medicament having an adjuvant activity in the treatment of type I or II diabetes by pancreatic islet transplantation.

As it will be demonstrated in the experimental section, the adjuvant activity of the microvesicles consists in the improvement of survival and functionality of the transplanted pancreatic islets and endothelial cells.

Moreover, as mentioned above, the adjuvant activity of MVs is not abolished by the administration of therapeutic doses of rapamycin as an immunosuppressor. Consequently, the MVs employed in the present invention are used as an adjuvant agent within the frame of pancreatic islet transplantation optionally in combination with rapamycin. The expression "in combination with" neither means that MVs and rapamycin must necessarily be mixed together, nor that they must necessarily be administered simultaneously. The expression "in combination with" simply means that the MVs are administered to the islet transplant recipient, preferably together with the islets themselves and generally by intravenous infusion, within the frame of a pancreatic islet transplantation procedure including at least one step in which the recipient is subjected to rapamycin-based immunosuppression before and/or during and/or after transplantation. In such a context, rapamycin is generally used to reach plasmatic through levels of 12-15 ng/ml in the first week after human islet transplantation (1).

As mentioned above, the MVs may be administered by intravenous infusion and they are generally administered together with the pancreatic islets. The portal vein is the preferred infusion site. Pancreatic islets are typically pre-incubated with MVs, before being infused into the recipient. A suitable MV dose to be administered depends on a plurality of factors, but it is generally comprised between 0.1 to 10 micrograms/Kg recipient body weight for the human being, preferably 1-5 micrograms/Kg.

The present invention is useful in a method of endothelial cell transplantation, comprising administering microvesicles (MVs) derived from an endothelial progenitor cell (EPC), to a subject which is in need of such treatment. Preferably, the subject is a human being. A major benefit resulting from the administration of MVs is that the survival and functionality of the transplanted endothelial cells are improved.

The present invention is also useful in a method of treating type I and II diabetes by pancreatic islet transplantation, comprising administering microvesicles (MVs) derived from a cell of the endothelial cell lineage, preferably from an endothelial progenitor cell (EPC), to a subject which is in need of such treatment. Preferably, the subject is a human being. A major benefit resulting from the administration of MVs is that the insulin production by the β-cells of the pancreatic islets is improved and that engraftment of the islets is improved. In order to treat type I and II diabetes by pancreatic islet transplantation, the islets are transplanted into the recipient's liver.

The following experimental section is provided by way of illustration only.

### MATERIALS AND METHODS

### Human islet and endothelial cell isolation

Ten different preparations of freshly purified human islets discarded from transplant use for inadequate islet mass were prepared following the Ricordi method (3). Purified islets (>90% pure) were cultured in CMRL medium (Mediatech Inc., Herndon, VA) containing 5 mg/mL albumin (Kedrion Spa, Lucca, Italy) and 2 mM glutamine (GIBCO BRL, Gaithersburg, MD).

Human pancreatic islet endothelial cell lines (IEC) were generated as follows. Briefly, cells outgrowing from islets were removed by trypsin/EDTA treatment and transfected with 4 mg pBR322 plasmid vector containing SV40-T large antigen gene at 250 mV and 960 mF in 4-mm electroporation cuvettes in an electroporator II (Invitrogen Corp., Carlsbad, CA). Clones were selected for 1 mg/mL G418 resistance and screened for immunofluorescence and FACS expression of endothelial markers. Positive clones were further subcloned by limiting dilution method and cultured in RPMI (Sigma), containing 10% FCS (Hyclone, Logan, Utah), 2 mM glutamine (GIBCO BRL) and endothelial growth factors (10 ng/mL VEGF, 10 ng/mL bFGF, 10 ng/mL PDGF and 0.5 U/mL heparin).

### Human endothelial progenitor cell isolation

Human endothelial progenitor cells (EPCs) were isolated from PBMC of healthy donors by density centrifugation. Purified cells were plated on fibronectin-coated culture flasks in a medium supplemented with 5% FCS and endothelial growth factors (10 ng/mL VEGF, 10 ng/mL bFGF, 10 ng/mL PDGF and 0.5 U/mL heparin) and characterized as previously reported (8). EPCs from 5-10 passages were used in this study.

### Isolation and characterization of microvesicle-s (MVs) from EPCs

MVs were obtained from supernatants of EPCs as previously described (Deregibus et al., Blood, 2007). Briefly, after centrifugation at 2,000 g for 20 minutes to remove debris, cell-free supernatants were centrifuged at 100,000 g (Beckman Coulter Optima L-90K ultracentrifuge) for 1 hr at 4 °C, washed in serum-free medium 199 containing 25mM N-2- hydroxyethylpiperazine-N'-2-ethanesulfonic acid (HEPES) (Sigma-Aldrich) and submitted to a second ultracentrifugation in the same conditions. In selected experiments. EPC-derived MVs were labeled with the red fluorescent aliphatic chromophore PKH26 dye (Sigma Aldrich). After labeling, MVs were newly washed by ultracentrifugation at 100,000 g for 1 hr at 4 °C. MV pellets were re-suspended in medium 199 and the protein content was quantified by the Bradford method (BioRad, Hercules, CA, USA). MV characterization was performed by microarray, FACS analysis, scanning and transmission electron microscopy as previously reported (Deregibus et al., Blood, 2007). MVs were stored at -80°C until use.

### Internalization of EPC-derived MVs in human islets and IEC

Human islets (500 IEQ) were cultured for 6 hrs in the Rotary cell culture system in the presence of 10 µg/ml EPC-derived MVs labeled with the red fluorescent dye PKH26 (Sigma). MV internalization was evaluated by confocal microscopy (Deregibus et al., Blood, 2007). IEC were cultured in 24-well plates in the presence of vehicle alone or 10 µg/ml labeled EPC-derived MVs. In selected experiments 10 µg/ml blocking antibodies directed to αvβ3-integrin (BioLegend), α4-integrin, α5-integrin (Chemicon Int.), CD29 (Becton Dickinson Biosciences) or L-selectin (Pharmingen) were added to MV-stimulated IEC. MV internalization in IEC was evaluated by confocal microscopy and FACS analysis.

### Assessment of insulin secretory response and viability

Islet function was evaluated by ELISA insulin secretory response (ALPCO Windham, NH). Briefly, islets incubated with after pre-incubation for 1 hr in 2.8 mM glucose medium followed by 2-hr incubation in 25 mM glucose medium. The stimulation indices were calculated as ratio between insulin secretion (mU/L/IEQ) in the presence of high glucose medium and mean basal insulin secretion levels using a spectrophotometric plate reader at 590-nm wave length. Islet viability was assessed by dual staining with 0.46 µM fluoresceine diacetate and 14.34 µM propidium iodide (both from Sigma Aldrich, St. Louis, MO).

### Caspase-3 ELISA

The activity of caspase-3 was assessed by ELISA (Chemicon, Temecula, CA) based on the spectrophotometric detection of the cromophore p-nitroanilide (pNA) after cleavage from the labelled substrate DEVD-pNA, that is recognized by caspases. Islet lysates were diluted with an appropriate reaction buffer and DEVD-pNA was added at a final concentration of 50M. Samples were analyzed in an automatized ELISA reader at a wave length of 405 nm. Each experiment was performed in triplicate.

### Endothelial outgrowth from freshly purified islets

Freshly purified islets (500 IEQ) were plated on tissue culture dishes and incubated with normal medium in the presence or absence of EPC-derived MVs. In selected experiments, therapeutic doses of rapamycin (10 ng/ml) were added to EPC-derived MVs. A medium containing endothelial growth factors was used as positive control for cell outgrowth. Endothelial outgrowth from islets was studied under a Nikon microscope system for living cell analysis. The same experimental procedures were performed on five different preparations of freshly purified islets.

### Migration of IEC

IEC were plated and rested for 12 hrs with RPMI containing 1% FCS and subsequently incubated with different stimuli. Cell migration was studied with a 10× phase-contrast objective under the above-mentioned Nikon system. The net migratory speed (velocity straight line) was calculated by the MicroImage software based on the straight line distance between the starting and ending points divided by the time of observation. Migration of at least 30 cells for each experimental point was analyzed.

### IEC viability assay

IEC were cultured on 24-well plates (Falcon Labware, Oxnard, CA) at a concentration of 5 × 10⁴ cells/well, starved for 12 hrs without FCS and then incubated with increasing doses of EPC-derived MVs (1-50 µg/ml) in a medium without phenol red containing 250 µg/mL XTT (Sigma Aldrich). In selected experiments, therapeutic doses of rapamycin (10 ng/ml) were added to EPC-derived MVs. A medium containing endothelial growth factors without EPC-derived MVs was used as positive control The absorption values were determined at 450 nm wave length. All experiments were performed in triplicate.

### Detection of IEC apoptosis

IEC were subjected to TUNEL assay (terminal deoxynucleotidyltransferase (TdT)-mediated dUTP nick end labeling) (ApopTag, Oncor, Gaithersburg, MD) after starving for 12 hrs without FCS and subsequent incubation for 48 hrs in the presence or absence of EPC-derived MVs. In selected experiments, therapeutic doses of rapamycin (10 ng/ml) were added to EPC-derived MVs. After incubation, cells were fixed in 1% paraformaldehyde, post-fixed in pre-cooled ethanol-acetic acid 2:1, incubated with TdT enzyme in a humidified chamber at 37° C for 1 hr and counterstained with antidigoxigenin-FITC antibody and with propidium iodide (1 µg/mL). Samples were analyzed under a UV light microscope with an appropriate mounting medium. Green-stained apoptotic cells were counted in different microscopic fields (magnification xl 00).

### In vitro angiogenesis assay

*In vitro* formation of capillary-like structures was studied on 500 IEQ human islets or on IEC-GFP (5 x 10⁴ cells/well) seeded on growth factor-reduced Matrigel (Becton Dickinson, Bedford, MA) diluted 1:1 in ice with cold DMEM (Sigma Aldrich). Cells were observed under a Nikon-inverted microscope, using a 10x/0.25 NA objective lens, and experimental results were recorded after 6-hr incubation with different stimuli at 37°C. Image analysis was performed at 1-hr intervals by the MicroImage analysis system (Casti Imaging). Results are given as average number of capillary-like structures/field (magnification x100) ± SD of three different experiments.

### Xenografts in SCID mice

Subcutaneous (s.c.) implantation of islets or IEC-GFP in Matrigel plugs was performed to evaluate the angiogenic effects of EPC-derived MVs *in vivo.* Briefly, Matrigel was mantained at -20.C until use and thawed at 4° C overnight immediately before implant. Freshly purified islets (2000 IEQ), or IEC-GFP (10⁴ cells) were resuspended in 250 µL of fresh medium without FCS and mixed to 500 µL of Matrigel on ice using cooled pipette tips in the absence or in the presence of 10 µg/ml EPC-derived MVs and s.c. implanted into the scruff region of the neck of SCID mice. After 2 weeks mice were sacrificed and Matrigel plugs were retrieved for histology and immuno-histochemistry as reported below. Six animals for each experimental group were examined.

### Gene array technology

Human GEarray kit for the study of angiogenesis markers (SuperArray Inc., Bethesda, MD) was used to characterize the gene expression profiles of IEC incubated with vehicle alone or 10 µg/ml EPC-derived MVs for 48 hrs. Hybridization was performed according to the manufacturer's instructions.

### Immunofluorescence studies

Freshly purified human islets or IEC cultured in chamber slides in different experimental conditions were fixed with 1% paraformaldehyde, permeabilized with 0.1 % Triton-X-100 (Sigma) when needed and stained for 1 hr with a polyclonal rabbit anti-human insulin antibody or with the following antibodies directed to endothelial antigens: anti-human CD31 (PECAM-1), anti-human tie-2 and anti-human VEGF-R2 (KDR) (all from Santa-Cruz Biotechnology, Santa Cruz, CA), mouse monoclonal anti-human VEGF (US Biological, Swampscott, MA), mouse monoclonal anti-human αVβ3-integrin (Chemicon International, Temecula, CA), rabbit polyclonal anti-human von Willebrand factor (vWF) or Alexa Fluor-conjugated acetylated-LDL (all from Invitrogen, Carlsbad, CA). All samples were incubated with appropriate Alexa Fluor-conjugated secondary antibodies (Invitrogen) for 30 minutes. Matrigel implants containing human islets were fixed in formaldehyde and embedded in paraffin prior to staining. All samples were counterstained with 1 mg/mL propidium iodide or with 0.5 mg/mL Hoechst, mounted with antifade mounting medium (Vector Laboratories, Burlingame, CA), and examined by fluorescence microscopy. The evaluation of intra-islet revascularization and MV internalization was performed by confocal microscopy (Leica TCS SP2 Heidelberg, Germany) after co-staining for insulin and for the above mentioned endothelial markers. The MicroImage software was used to determine the number and the total area/section of neoformed vessels within islets.

### FACS analysis

Unstimulated or stimulated IEC were detached from tissue culture plates with EDTA and stained for 45 min at 4.C with FITC-, PE-conjugated antibodies or red fluorescent-labeled EPC-derived MVs. Cells were then fixed in 1% paraformaldehyde and subjected to FACS analysis (Becton Dickinson, Mountain View, CA).

### Western blot analysis

IEC cultured in different experimental conditions were lysed at 4 °C for 1 hr in a lysis buffer (50 mM Tris-HCl, pH 8.3, containing 1% Triton X-100, 1 mM PMSF, 10 µg/ml leupeptin, and 100 units/ml aprotinin). Aliquots of the cell lysates containing 30 µg of protein, as determined by Bradford method, were subjected to 4-15% gradient SDS-PAGE under reducing conditions and electroblotted onto nitrocellulose membrane filters. The following primary antibodies were used: monoclonal antibody directed to Akt (Upstate, Charlottesville, VI, USA), phospho-Akt and rabbit polyclonal antibody against phospho-eNOS (Cell Signalling, Beverly, MA, USA), mouse monoclonal antibody against actin, mouse monoclonal anti-Bcl-xL and rabbit polyclonal antibody against eNOS (Santa Cruz).

### Lymphocyte adhesion to IEC monolayers

PBMC were isolated from healthy volunteers by density gradient and labeled overnight with 10 µm Vybrant Cell Tracer kit (Invitrogen) according to manufacturer's instructions in RPMI and 10% FBS. Labeled cells were counted, re-suspended to 50 × 10⁶/mL in RPMI without FCS and added to confluent monolayer of IEC cultured on six-well plates and previously incubated with vehicle alone or inflammatory cytokines (10 ng/mL TNF-alpha and 10 ng/mL IFN-gamma) in the presence or in the absence of 10 µg/mL EPC-derived MVs. Experiments were carried out in triplicate for 1 hr at 37.C in conditions of slight agitation. At the end of incubation, plates were filled with medium and aspirated three times to remove unbound cells. All samples were fixed with 1% paraformaldehyde and observed by fluorescence microscopy. Green fluorescent cells were counted on 10 different fields at x 200 magnification.

### Statistical analysis

All data of different experimental procedures are expressed as average ± SD. Statistical analysis was performed by Student's t-test or ANOVA with Newmann-Keuls multicomparison test where appropriated.

### RESULTS

### Characterization of EPC-derived MVs

Scanning electron microscopy and FACS analysis showed the presence of spheroid MVs in pellets derived from ultracentrifugation of EPC supernatants. The majority of EPC-derived MVs sized approximately 1 µm and expressed several molecules usually found on the EPC surface such as intracellular adhesion molecule-1 (ICAM-1), α4 integrin, CD29 (β1 integrin) and CD44. Moreover, we also found on the EPC-derived MV's surface, the presence of CD62L (L-selectin), a protein essential for EPC's homing in injured tissues.

### EPC-derived MVs induced endothelial outgrowth from islets

In comparison to incubation with vehicle alone, 10 µg/ml EPC-derived MVs induced cell outgrowth from islet surface detectable after 24 hrs and more evident after 96 hrs. Cells outgrowing from islets were characterized as endothelial cells by specific immunostaining with typical endothelial markers such as KDR (VEGFR-2), CD31 (PECAM-), von Willebrand Factor, CD 105 and nestin. Moreover, outgrowing cells showed the ability to internalize acetylated- LDL and to form capillary-like structures when seeded on Matrigel-coated plates.

### Rapamycin did not abolish endothelial outgrowth from islets induced by EPC-derived MVs

As we previously reported (7), therapeutic doses of rapamycin (10 ng/ml) abrogated endothelial outgrowth induced by incubation of islets with an endothelial growth factor-enriched medium. By contrast, the same dose of rapamycin did not completely abolish endothelial outgrowth induced by EPC-derived MVs, suggesting the involvement of mechanisms other than growth factor stimulation in this angiogenic process.

### EPC-derived MVs enhanced insulin secretion, preserved islet viability and decreased caspase-3 activity

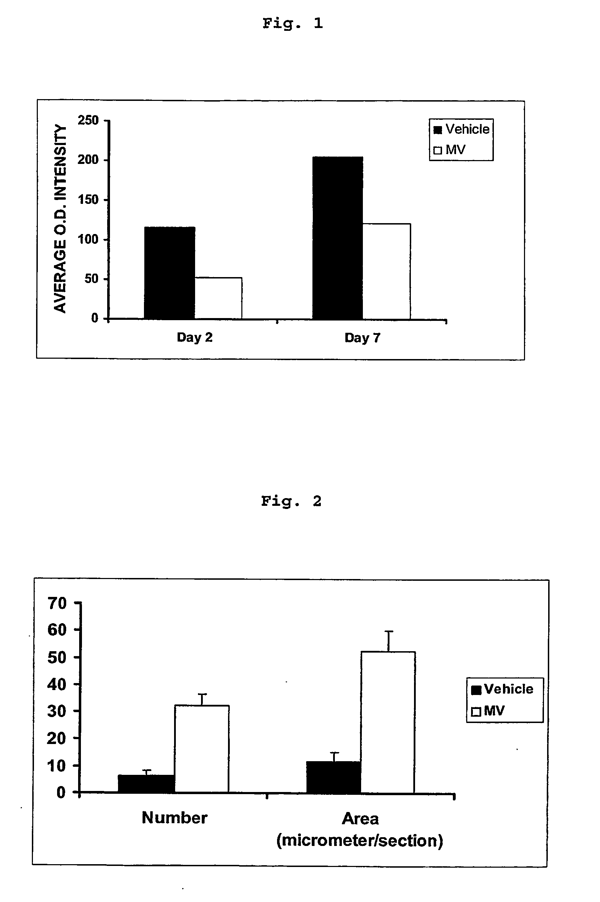

Islet function, evaluated as insulin response after high glucose challenge, was significantly higher in the presence of EPC-derived MVs with respect to vehicle alone after 2 and 7 days of culture. In addition, dual staining with fluorescein diacetate and propidium iodide evidenced a sustained islet viability in the presence of EPC-derived MVs. The inhibition of islet apoptosis induced by EPC-derived MVs was further confirmed by the significant decrease in caspase-3 activity observed in islet lysates after 2 and 7 days of incubation with EPC-derived MVs (Fig. 1).

### EPC-derived MVs are internalized in beta cells and islet endothelium

EPC-derived MV internalization in human islets was evaluated after staining of the MVs with the red fluorescent dye PKH26. Confocal microscopy analysis showed the presence of labeled-MVs into both beta-cells and islet endothelium detected by co-staining with insulin, GLUT-2 or with the endothelial markers CD31, KDR and von Willebrand Factor. In addition, EPC-derived MVs were also incorporated by different lines of islet-derived endothelial cells (IEC) after incubation for 30 minutes at 37° C as shown by confocal microscopy micrographs and FACS analysis. To identify the role of selective molecules involved in MV internalization, EPC-derived MVs were pre-incubated for 15 minutes at 4° C with different blocking antibodies. As previously reported for other endothelial cell lines, also in islet endothelium the presence of α4 integrin , CD29 and in addition L-selectin is essential for MV internalization into target cells.

### EPC-derived MVs increase neoangiogenesis of human islets implanted subcutaneously into Matrigel plugs in SCID mice

The effect of EPC-derived MVs on islet neoangiogenesis was evaluated *in vivo* after subcutaneous injection of freshly purified islets within Matrigel plugs into the scruff region of the neck of SCID mice, in a xenograft model previously described (7). In the presence of EPC-derived MVs, implants showed a marked increase of vascular density within islets as detected by hematoxylin-eosin staining and by immunoistochemistry analysis of the endothelial markers KDR and CD31. Islets treated with EPC-derived MVs also presented a diffuse staining for insulin. In addition, the evaluation of total number and area of neoformed vessels within Matrigel sections confirmed a significant increase of angiogenesis in islets stimulated with EPC-derived MVs. Figure 2 shows the count of the total number and area (expressed as micrometer²/section) of islet xenografts in SCID mice in the presence or in the absence of EPC-derived MVs.

### EPC-derived MVs enhance in vitro and in vivo IEG-GFP angiogenesis

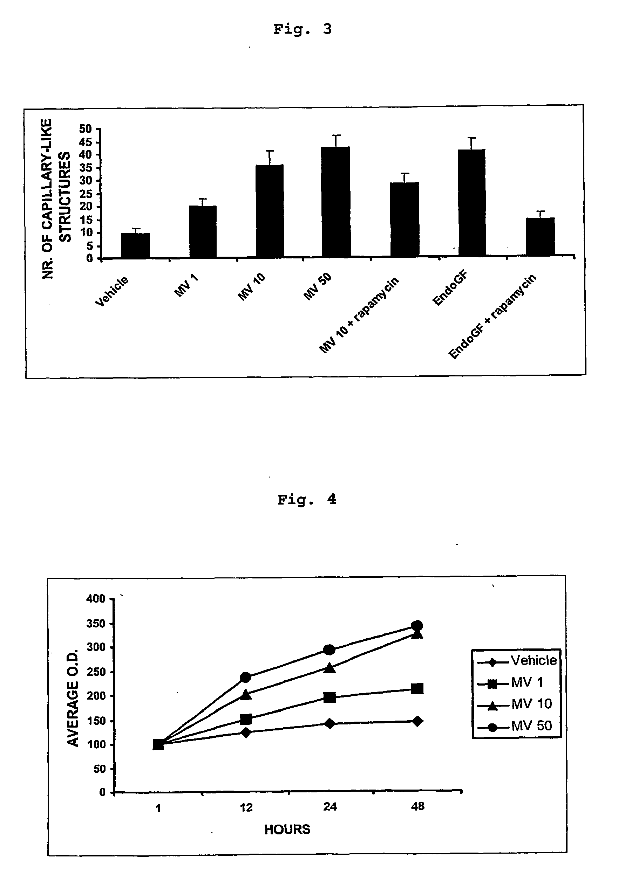

We evaluated the modulation of *in vitro* angiogenesis induced by EPC-derived MVs on IEC transduced by a lentiviral vector expressing GFP (IEC-GFP). When seeded on Matrigel-coated surfaces, IEC-GFP spontaneously formed capillary-like structures. The addition of EPC-derived MVs accelerated the angiogenic process, resulting in a dose-dependent enhanced formation of an organized capillary network. Rapamycin (10 ng/ml) did not completely abrogate the angiogenic effect of EPC-derived MVs. Fig. 3 shows the results obtained, i.e. the dose-dependent effect of EPC-derived MVs on IEC *in vitro* angiogenesis and the effect of rapamycin on MV-induced angiogenesis (EndoGF = medium enriched with endothelial growth factors).

We then performed xenografts of IEC-GFP by injection into the scruff region of the neck of SCID mice as mentioned above. Consistently with the *in vitro* angiogenesis results, IEC showed a marked enhancement of their ability to proliferate and to form neovessels in the presence of EPC-derived MVs, as detected by histologic and Immunofluorescence analysis. Moreover, EPC-derived MVs induced a significant increase in the total number and area of the vessels in the Matrigel sections examined.

### EPC-derived MVs exert a proliferative, anti-apoptotic and migratory effect on IEC

We evaluated the effect of EPC-derived MVs on islet endothelium growth. IEC were starved overnight without FCS and subsequently incubated with increasing doses of EPC-derived MVs. EPC-derived MVs induced a significant dose-dependent increase of IEC proliferation. The proliferative effect was detectable at doses of 1 µg/mL and reached a plateau at the dose of 50 µg/mL. In addition, we found that IEC challenged with EPC-derived MVs showed an enhanced resistance to apoptosis induced by serum deprivation. The anti-apoptotic effects exerted by EPC-derived MVs on IEC were not completely abolished by co-incubation with 10 ng/ml rapamycin. Fig. 4 shows the dose-dependent proliferative effect induced by increasing doses of EPC-derived MVs on IEC.

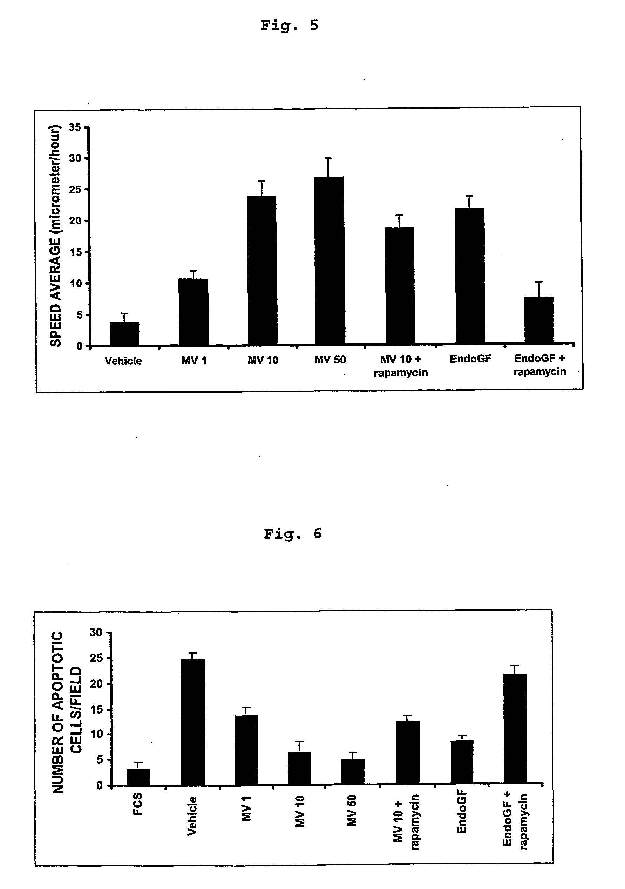

The effects of EPC-derived MVs on IEC migration, an index of endothelial cell activation, were then studied by time-lapse recording microscopy. The baseline migration rate of IEC corresponding to the spontaneous motility of resting cells was found to remain stable over the whole period of observation, never exceeding 5-6 m/hr. EPC-derived MVs induced a significant dose-dependent increase in spontaneous cell motility. Consistent with apoptosis data, 10 ng/ml rapamycin did not entirely block IEC migratory activity. Fig.5 shows the dose-dependent migratory effect of EPC-derived MVs on IEC cultured in serum deprivation conditions and the effect of rapamycin on MV-induced motility. (EndoGF= medium. enriched with endothelial growth factors).

Figure 6 shows the dose-dependent anti-apoptotic effect of EPC-derived MVs on IEC cultured in serum deprivation condition and the effect of rapamycin on MV-induced rescue from apoptosis (EndoGF= medium enriched with endothelial growth factors).

### Pathways involved in IEC angiogenesis induced by EPC-derived MVs

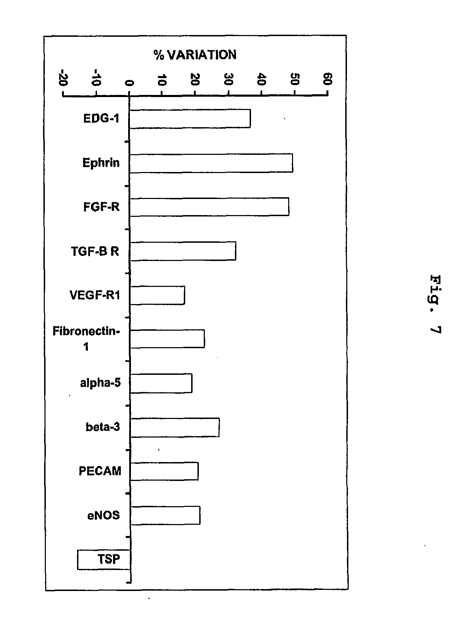

We investigated, both at the gene and at the protein level in IEC, the modulation of the expression of molecules involved in angiogenesis after incubation with EPC-derived MVs. In IEC stimulated with EPC-derived MVs, the gene array analysis revealed the enhanced expression of the Endothelial Differentiation-related Factor-1 (EDF-1), of the tyrosine kinase receptor ephrin, of other different growth factor receptors (FGF-R, VEGF-R1, TGFβ-R), of pro-angiogenic integrins (α5 and β3) and matrix molecules (fibronectin-1), of specific endothelial markers (CD31) and eNOS (Fig. 7). In addition, IEC stimulated with EPC-derived MVs showed the down-regulation of the anti-angiogenic factor thrombospondin-1. By immunofluorescence or western blot analysis, we confirmed that EPC-derived MVs modulate in islet endothelium molecules involved in angiogenesis and cell survival such as Akt/P-Akt, Bcl-xL and eNOS.

### Lymphocyte adhesion to hIEC

We evaluated the role of EPC-derived MVs in endothelial-lymphocyte interaction. The addition of 10 µg/ml EPC-derived MVs significantly inhibited spontaneous lymphocyte adhesion to IEC monolayers in condition of slight agitation. The inhibition of lymphocyte adhesion was particularly evident in the presence of a pro-inflammatory microenvironment obtained after incubation of IEC monolayers with 10 ng/mL TNF-alpha and 10 ng/mL IFN-gamma.

### DISCUSSION

In this study, we demonstrate that EPC-derived MVs promote angiogenesis and insulin secretion *in vitro* and in an experimental model of subcutaneous islet transplantation in Matrigel plugs into SCID mice. In addition, EPC-derived MVs sustain *in vitro* proliferation, resistance to apoptosis, migration, formation of capillary-like structures and *in vivo* angiogenesis of islet-derived endothelial cell lines.

We also demonstrate that EPC-derived MVs are internalized both in beta cells and in islet endothelium, sustaining insulin secretion and *in vitro* angiogensis through a possible paracrine effect. Moreover, EPC-derived MVs induce a significant increase in the total number and area of neoformed vessels in freshly purified human islets xenotransplanted in Matrigel plugs into the scruff region of the neck of SCID mice. These results suggest a direct activation of islet endothelium angiogenesis induced by EPC-derived MVs. In addition, the internalization of EPC-derived MVs in islet endothelial and β-cells may promote a further release of paracrine factors from both cell types capable of sustaining survival in detrimental isolation and culture conditions.

MV uptake is mediated by specific cell membrane proteins such as α4, CD29 and L-selectin. In leukocyte biology, different adhesion receptors regulate their interaction with endothelial cells through rolling and subsequent extravasation into inflammatory sites. The selectin receptor family plays a key role in the early events of vascular adhesion. We recently demonstrated that EPCs express L-selectin on their surface and that this molecule is essential for EPC homing into sites of vascular injury. In this study we show that also EPC-derived MVs internalize into target cells via an L-selectin-mediated mechanism thanks to the binding to fucosylated residues or other oligosaccharidic ligands usually up-regulated in tissue exposed to ischemia-reperfusion injury.

We have also found that EPC-derived MVs enhance *in vitro* proliferation, resistance to apoptosis induced by serum deprivation, migration of islet endothelial cell lines and endothelial outgrowth from islets. Interestingly, all of these biological phenomena induced by EPC-derived MVs are not completely abolished by co-incubation with therapeutic doses of rapamycin, whereas the same dose of this pharmacological agent completely inhibits the trophic effects exerted on IEC by soluble growth factors added to culture medium. These results suggest a putative horizontal mRNA transfer between EPC-derived MVs and islet endothelium, that is confirmed by the significant inhibition of MV-induced effects on IEC after their pre-incubation with RNase. In addition, gene array analysis of molecules involved in angiogenesis showed an increased expression of mRNA carried by EPC-derived MVs such as Bcl-xL and eNOS. EPC-derived MVs trigger the activation of PI3K/Akt signaling pathways and eNOS in IEC.

EPC-derived MVs also induce the up-regulation of ephrins and EDF-1 in IEC. Ephrins and their relative tyrosine kinase receptors are deeply involved in cell motility and adhesion during blood-vessel-wall assembly and induce endothelial cell chemotaxis and branching remodeling. By phage display and laser microdissection, ephrin family members and their receptors have been identified in islet endothelium (9). EDF-1 is a low molecular weight polypeptide down-regulated in human endothelial cells undergoing differentiation, quiescence and senescence. Our findings, suggest that EPC-derived MVs activate a de-differentiative and proliferative program in IEC. In addition, in comparison to vehicle alone, IEC stimulated with EPC-derived MVs show increased levels of CD31 (PECAM-1), a molecule known to inhibit endothelial apoptosis and down-regulation of thrombospondin-1 (TSP_1). TSP-1 is an inhibitor of angiogenesis also promoting apoptosis in activated endothelial cells. We have recently shown that TSP-1 is up-regulated in IEC in response to rapamycin. Moreover, it has been shown that TSP-1 knock out mice present islet hyperplasia characterized by an increased blood vessel density (10).

Another finding resulting from the present study is that in the presence of EPC-derived MVs, lymphocyte show decreased adhesion properties to IEC monolayer. Islet endothelial cells play a key role not only in revascularization of transplanted islets, but also in mechanisms related to allo- and autoimmunity. Indeed, the activation of the immune response is another important cause of islet graft loss. It has been previously shown that IEC are antigen presenting cells able to acquire insulin secreted by beta-cells thus contributing to the specificity of homing of activated T lymphocytes into naive and transplanted islets. We found that, in contrast to exosomes derived from other cell types, EPC-derived MVs do not present MHC antigens on their surface. This finding, together with the significant reduction in lymphocytes to IEC monolayers, suggests a possible anti-inflammatory action of EPC-derived MVs, that may limit the triggering of allo- and autoimmunity in transplanted islets.

In conclusion, the results of our study demonstrate that chimerism between islets and EPC promotes beta-cell function and angiogenesis on islets through a paracrine mechanism mediated by the release of activated MVs from cell surface. The angiogenic properties of EPC-derived MVs were obtained also in the presence of rapamycin at doses usually adopted in clinical islet transplantation. The easy collection of EPCs from peripheral blood indicate them as a potential therapeutic option to improve islet revascularization after transplantation. Moreover, even though further experiments are needed to investigate the metabolic benefit of this therapeutic approach on beta-cell function, the use of EPC-derived MVs may offer a temporary limited switch on mechanism of angiogenesis without the detrimental effects exerted by the infusion of whole cells.

### REFERENCES

1. Shapiro AM, Lakey JR, Ryan EA et al. Islet transplantation in seven patients with type 1 diabetes mellitus using a glucocorticoid-free immunosuppressive regimen. N Engl J Med 2000; 343: 230-238.
2. Biancone L, Ricordi C. Pancreatic islet transplantation: An update. Cell Transplant 2002; 11: 309-311.
3. Ricordi C, Lacy PE, Scharp DW. Automated islet isolation from human pancreas. Diabetes 1989; 38(Suppl 1): 140-142.
4. Mathews V, Hanson PT, Ford E, Fujita J, Polonsky KS, Graubert TA. Recruitment of bone marrow-derived endothelial cells to sites of pancreatic beta-cell injury. Diabetes. 2004 Jan;53(1):91-8.
5. Contreras JL, Smyth CA, Eckstein C, Bilbao G, Thompson JA, Young CJ, Eckhoff DE. Peripheral mobilization of recipient bone marrow-derived endothelial progenitor cells enhances pancreatic islet revascularization and engraftment after intraportal transplantation. Surgery. 2003 Aug;134(2):390-8.
6. Urbich C, Dimmeler S. Endothelial progenitor cells functional characterization. Trends Cardiovasc Med. 2004 Nov;14(8):318-22.
7. Cantaluppi V, Biancone L, Romanazzi GM, Figliolini F, Beltramo S, Ninniri MS, Galimi F, Romagnoli R, Franchello A, Salizzoni M, Perin PC, Ricordi C, Segoloni GP, Camussi G. Antiangiogenic and immunomodulatory effects of rapamycin on islet endothelium: relevance for islet transplantation. Am J Transplant. 2006 Nov;6(11):2601-11.
8. Biancone L, Cantaluppi V, Duo D, Deregibus MC, Torre C, Camussi G. Role of L-selectin in the vascular homing of peripheral blood-derived endothelial progenitor cells. J Immunol. 2004 Oct 15;173(8):5268-74.
9. Yao VJ, Ozawa MG, Trepel M, Arap W, McDonald DM, Pasqualini R. Targeting pancreatic islets with phage display assisted by laser pressure catapult microdissection. Am J Pathol. 2005 Feb;166(2):625-36.
10. Li X, Zhang L, Meshinchi S, Dias-Leme C, Raffin D, Johnson JD, Treutelaar MK, Burant CF. Islet microvasculature in islet hyperplasia and failure in a model of type 2 diabetes. Diabetes. 2006 Nov;55(11):2965-73.

## Claims

1. A microvesicle (MV) derived from an endothelial progenitor cell (EPC) for use as a medicament having adjuvant activity in the treatment of type I and II diabetes by pancreatic islet transplantation.

2. The microvesicle (MVs) for use according to claim 1, which is in the form of a medicament suitable for administration by intravenous infusion.

3. The microvesicle (MV) for use according to claim 2, wherein the medicament is suitable for administering a microvesicle dose comprised between 0.1 and 10 micrograms/Kg recipient weight.

4. The microvesicle (MV) for use according to any of claims 1 to 3, wherein the pancreatic islet transplantation procedure includes at least one step in which the recipient is subjected to rapamycin-based immunosuppression before and/or during and/or after transplantation.

## Patentansprüche

1. Mikrovesikel (MV), das aus einer endothelialen Vorläuferzelle (EPC) stammt, für die Verwendung als ein Medikament mit adjuvanter Aktivität in der Behandlung von Diabetes von Typ I und II durch Pankreas-Inselzelltransplantation.

2. Mikrovesikel (MV) für die Verwendung nach Anspruch 1, welches in der Form eines Medikaments ist, das für die Verabreichung durch intravenöse Infusion geeignet ist.

3. Mikrovesikel (MV) für die Verwendung nach Anspruch 2, wobei das Medikament für die Verabreichung einer Dosis Mikrovesikel geeignet ist, die zwischen 0,1 und 10 Mikrogramm/kg des Empfängergewichts umfasst.

4. Mikrovesikel (MV) für die Verwendung nach einem der Ansprüche 1 bis 3, wobei das Pankreas-Inselzelltransplantationsverfahren wenigstens einen Schritt einschließt, in welchem der Empfänger einer Immunosuppression auf Grundlage von Rapamycin vor und/oder während und/oder nach der Transplantation unterzogen wird.

## Revendications

1. Microvésicule (MV) dérivée d'une cellule progénitrice endothéliale (CPE) destinée à être utilisée comme médicament ayant une activité d'adjuvant dans le traitement des diabètes de type I et II par transplantation d'îlots pancréatiques.

2. Microvésicule (MV) destinée à être utilisée selon la revendication 1, qui est sous forme d'un médicament approprié à l'administration par perfusion intraveineuse.

3. Microvésicule (MV) destinée à être utilisée selon la revendication 2, où le médicament est approprié pour l'administration d'une dose de microvésicules comprise entre 0,1 et 10 microgrammes/kg de poids du receveur.

4. Microvésicule (MV) destinée à être utilisée selon l'une quelconque des revendications 1 à 3, où le processus de transplantation d'îlots pancréatiques inclut au moins une étape dans laquelle le receveur est soumis à une immunosuppression basée sur la rapamycine avant et/ou pendant et/ou après la transplantation.
